# EUROPÄISCHE PATENTSCHRIFT

(11) **EP 4 601 587 B1**
(45) Veröffentlichungstag und Bekanntmachung des Hinweises auf die Patenterteilung: **18.02.2026**
(21) Anmeldenummer: 23786518.3
(22) Anmeldetag: 05.10.2023
(51) Int. Cl.: A61F 2/70, A61F 2/66, A61F 2/68

(54) **VERFAHREN ZUM STEUERN EINER UNTERSCHENKELPROTHESE UND UNTERSCHENKELPROTHESE**
PROCEDURE FOR CONTROLLING A TRANSTIBIAL PROSTHESIS AND TRANSTIBIAL PROSTHESIS
PROCÉDÉ DE COMMANDE D'UNE PROTHÈSE DE JAMBE ET PROTHÈSE DE JAMBE

(30) Priorität: 10.10.2022 DE 102022126222
(43) Veröffentlichungstag der Anmeldung: 20.08.2025
(73) Patentinhaber: Otto Bock Healthcare Products GmbH, 1110 Wien (AT)
(72) Erfinder: BOHLAND, Andreas, 1110 Wien (AT); PAPPE, Alexander, 1110 Wien (AT); SEYR, Martin, 1110 Wien (AT)
(74) Vertreter: Gramm, Lins & Partner Patent- und Rechtsanwälte PartGmbB
(86) Internationale Anmeldenummer: PCT/EP2023/077557
(87) Internationale Veröffentlichungsnummer: WO 2024/078957

(56) Entgegenhaltungen:
- JP-A- 2013 070 785
- US-A1- 2013 310 949
- US-A1- 2018 008 434
- US-A1- 2018 147 074
- US-A1- 2022 304 831
- US-B2- 10 314 724

## Beschreibung

Die Erfindung betrifft ein Verfahren zum Steuern einer Unterschenkelprothese, die ein Fußelement, ein schwenkbar daran angeordnetes Unterschenkelelement und eine verstellbare Widerstandseinrichtung zum Aufbringen eines Widerstandes gegen eine Verschwenkung des Fußelementes relativ zu dem Unterschenkelelement aufweist. Die Erfindung betrifft zudem eine Unterschenkelprothese, die mittels eines solchen Verfahrens steuerbar ist.

Ein Verfahren zum Betreiben einer Unterschenkelprothese ist beispielsweise aus der US 2022/304831 A1 und der US 2013/0310949 A1 bekannt. Die US 10 314 724 B2 und die US 2018/0008434 A1 beschreiben jeweils prothetische Fußelemente, die mit einem Unterschenkelelement gemeinsam eine Unterschenkelprothese bilden können. Die Prothesenfüße weisen jeweils ein Knöchelgelenk auf, das hydraulisch gedämpft sein kann. Dokument US 2022/304831 A1 wird als nächstliegender Stand der Technik betrachtet.

Aus der US 2018/0147074 A1 ist ein Stoßdämpfer für ein prothetisches Gelenk und aus der JP 2013/070785 A ein Exoskelett bekannt.

Unterschenkelprothesen der oben genannten Art können über die verstellbare Widerstandseinrichtung während eines Schrittzyklus unterschiedliche Widerstände gegen die Verschwenkung des Fußelementes relativ zum Unterschenkelelement erzeugen. Dies wird im Stand der Technik seit langem genutzt um das natürliche Gangbild zu imitieren. Wichtig ist dabei in der Regel, dass etwa in der Mitte der Standphase eines Schrittzyklus der Widerstand stark erhöht wird. Dies geht oft soweit, dass ab diesem Zeitpunkt eine weitere Verschwenkung des Fußelementes relativ zum Unterschenkelelement nicht mehr möglich ist. Die Standphase eines Schrittzyklus ist dabei dadurch definiert, dass das Fußelement Bodenkontakt hat.

Ab dem Moment, in dem der Widerstand erhöht wurde, ist eine weitere Verschwenkung des Fußelementes relativ zum Unterschenkelelement nicht oder nur noch erschwert möglich. Das Abrollen des Fußes erfolgt dann folglich über den Vorfuß, der im Wesentlichen durch die Zehen gebildet wird. Dadurch verändert sich die Hebellänge, über die abgerollt wird.

Aus dem Stand der Technik ist bekannt, den Zeitpunkt, zu dem der Widerstand erhöht wird davon abhängig zu machen, welche Steigung der Untergrund hat. Geht der Träger beispielsweise eine Rampe hinauf, sollte der Schaltzeitpunkt, zu dem der Widerstand erhöht wird, innerhalb des Schrittzyklus nach hinten, also zu einem späteren Zeitpunkt, verschoben werden.

Oftmals wird dabei ein Fußelement und/oder ein Unterschenkelelement verwendet, das mit einem Absolutwinkelsensor ausgerüstet ist, mit dem die Neigung des Fußelementes während der Standphase des Schrittzyklus bestimmt werden kann. So lässt sich auf einfache Weise ermitteln, ob der Träger beispielsweise eine schräge Ebene, also beispielsweise eine Rampe, hinaufgeht oder hinabgeht. Dieses Verfahren ist jedoch nicht anwendbar, wenn beispielsweise festgestellt werden soll, ob der Träger der Unterschenkelprothese eine Treppe hinaufgeht oder hinabgeht. Der Neigungswinkel des Fußelementes ist dabei unabhängig von der Bewegungsrichtung gleich, da die Treppenstufe horizontal ausgebildet ist. Das beschriebene Verfahren stößt zudem an seine Grenzen, wenn das Terrain, auf dem sich der Träger der Unterschenkelprothese bewegt, keine ebene Fläche bildet. Handelt es sich um eine unebene Fläche, lässt sich anhand der Neigung des Fußelementes nicht oder zumindest nicht immer verlässlich ermitteln, welche Steigung der Untergrund hat und wann der Schaltzeitpunkt sein sollte.

Aus der DE 10 2012 125 256 A1 ist es daher bekannt, zwei Schaltwinkel zu definieren. Der erste Schaltwinkel ist definiert als ein vorbestimmter Knöchelwinkelwert zwischen dem Unterschenkelelement und dem Fußelement. Sobald also der Knöchelwinkel, der zum Steuern der Unterschenkelprothese detektiert wird, diesen vorbestimmten Knöchelwinkelwert erreicht, gilt der erste Schaltwinkel als erreicht. Der zweite Schaltwinkel ist definiert als ein vorbestimmter Unterschenkelwinkelwert des Absolutwinkels des Unterschenkels. Unabhängig davon, welchen Wert der Knöchelwinkel zwischen dem Unterschenkelelement und dem Fußelement einnimmt, gilt der zweite Schaltwinkel als erreicht, wenn der Absolutwinkel des Unterschenkels den vorbestimmten Unterschenkelwinkelwert erreicht. Um nun die Unterschenkelprothese zu steuern muss festgelegt werden, welcher der beiden definierten Schaltwinkel der Winkel sein soll, zudem der Widerstand der Widerstandseinrichtung erhöht werden soll. Im Stand der Technik wird vorgeschlagen unabhängig von einer detektieren Lage des Fußelementes in der Standphase immer den Schaltwinkel zu verwenden, der innerhalb des jeweiligen Schrittzyklus als erster eintritt.

Dies ist in vielen Situationen eine gute Wahl, führt jedoch in manchen Situationen zu Problemen. Der erste Schaltwinkel kann beispielsweise als rechter Winkel, also 90°-Winkel definiert sein. Dies bedeutet, dass zwischen dem Unterschenkelelement und dem Fußelement, beispielsweise der Anlagefläche, die mit dem Boden in Kontakt kommt, ein rechter Winkel herrscht, wenn der erste Schaltwinkel definiert ist. Auch der zweite Schaltwinkel kann als 90°-Winkel definiert sein. Dies bedeutet, dass der zweite Schaltwinkel erreicht wird, wenn das Unterschenkelelement senkrecht steht, sich also parallel zur wirkenden Schwerkraft erstreckt.

Diese Definitionen der beiden Schaltwinkel führen beim Abwärtsgehen entlang einer Rampe dazu, dass zunächst der zweite Schaltwinkel erreicht wird. Der Widerstand wird folglich erhöht, wenn das Unterschenkelelement vertikal steht, was zu einem sicheren Stand führt, jedoch ein unnatürlich wirkendes Gangbild zur Folge hat. Beim Aufwärtsgehen entlang der Rampe führt diese Definition der beiden Schaltwinkel jedoch dazu, dass zunächst der erste Schaltwinkel erreicht wird. Dies ist beim Aufwärtsgehen jedoch zu früh und führt zu einem verfrühten Anstieg des von der Widerstandseinrichtung aufgebrachten Widerstandes.

Der Erfindung liegt daher die Aufgabe zugrunde, ein Verfahren zum Steuern einer Unterschenkelprothese so weiterzuentwickeln, dass ein sicheres und komfortables Gehen besser ermöglicht werden kann.

Die Erfindung löst die gestellte Aufgabe durch ein Verfahren zum Steuern einer Unterschenkelprothese, die ein Fußelement, ein schwenkbar daran angeordnetes Unterschenkelelement und eine verstellbare Widerstandseinrichtung zum Aufbringen eines Widerstandes gegen eine Verschwenkung des Fußelementes relativ zu dem Unterschenkelelement aufweist, wobei ein erster Schaltwinkel definiert ist als ein vorbestimmter Knöchelwinkelwert zwischen dem Unterschenkelelement und dem Fußelement und ein zweiter Schaltwinkel definiert ist als ein vorbestimmter Unterschenkelwinkelwert des Absolutwinkels des Unterschenkels. Bei dem Verfahren wird erfindungsgemäß zumindest auch aus einer Höhendifferenz zwischen der Position des Fußelementes in einem Schrittzyklus und der Position des Fußelementes im vorherigen Schrittzyklus bestimmt, ob der Träger der Unterschenkelprothese bergab geht, und der Widerstand der Widerstandseinrichtung auf einen vorbestimmten Widerstands wird erhöht wird, wenn ein Blockkriterium erfüllt ist. Erfindungsgemäß ist das Blockkriterium erfüllt, wenn bestimmt wurde, dass der Träger nicht bergab geht, und der zweite Schaltwinkel erreicht ist, oder wenn bestimmt wurde dass der Träger bergab geht, und der in dem Schrittzyklus später erreichte Schaltwinkel erreicht ist.

Anders als im Stand der Technik wird beim erfindungsgemäßen Verfahren nicht die Neigung des Fußelementes in der Standphase des Schrittzyklus benötigt. Stattdessen wird ermittelt, ob der Träger bergab geht, wobei die lokale Neigung des Untergrundes, die für die Neigung des Fußelementes in der Standphase ausschlaggebend ist, unberücksichtigt bleibt. Wenn die Bestimmung ergibt, dass der Träger nicht bergab geht, wird der Widerstand der Widerstandseinrichtung erhöht, wenn der zweite Schaltwinkel erreicht ist. Dies ist dann der Fall, wenn der Absolutwinkel des Unterschenkels den vorbestimmten Unterschenkelwinkelwert erreicht. Die Erhöhung des Widerstandes ist folglich in diesem Fall völlig unabhängig davon, wie die lokale Neigung des Bodens und damit auch die Neigung des Fußelementes in der Standphase, ist. Ergibt die Bestimmung jedoch, dass der Träger bergab geht, wird das Blockkriterium erreicht, wenn der in dem Schrittzyklus spätere Schaltwinkel erreicht ist.

Dadurch wird ein möglichst naturnahes Gangbild erzeugt, wenn zutreffend erkannt wird, ob der Träger der Unterschenkelprothese bergab geht oder nicht. Verfahren zum Steuern einer Unterschenkelprothese sollten jedoch vorzugsweise auch bei fehlerhafter Detektion oder Bestimmung einen sicheren Betrieb der Unterschenkelprothese gewährleisten, da der Träger der Unterschenkelprothese ansonsten stürzen und sich verletzen könnte. Wird beim erfindungsgemäßen Verfahren folglich fälschlicherweise erkannt, dass der Träger bergab geht, wird der im Schrittzyklus später erreichte Schaltwinkel verwendet, um den Widerstand der Widerstandseinrichtung zu erhöhen. Da der Träger in diesem Fall tatsächlich nicht bergab geht, sondern beispielsweise bergauf geht, ist dies der zweite Schaltwinkel, der als vorbestimmter Unterschenkelwinkelwert definiert ist. Es wird folglich auch bei der fehlerhaften Erkennung des bergab-Gehens der richtige Schaltwinkel verwendet.

Geht der Träger dagegen bergab und dies wird fälschlicherweise nicht erkannt, so wird als Blockkriterium der zweiten Schaltwinkel verwendet. Der Widerstand wird also erhöht, wenn der Absolutwinkel des Unterschenkels den vorbestimmten Unterschenkelwert erreicht. Dieser ist in der Regel jedoch der beim Bergabgehen in einem Schrittzyklus früher erreichte Schaltwinkel und somit für einen bergab gehenden Träger der falsche Schaltwinkel. Dies führt zwar nicht zum optimalen Gangbild, ermöglicht aber das stabile Stehen und ist somit kein wesentliches Sicherheitsproblem. Der Effekt dieser Fehlsteuerung auf den Träger ist insbesondere bei einem Fuß mit Carbonfeder ist relativ unproblematisch, da der Träger über den Vorfuß dank der Carbonfeder abrollen kann und so gebremst wird.

Vorzugsweise ist der vorbestimmten Widerstandswert so groß, dass eine weitere Verschwenkung des Fußelementes relativ zu dem Unterschenkelelement vollständig verhindert wird.

Vorzugsweise wird die Höhendifferenz zwischen der Position des Fußelementes in der Standphase der beiden Schrittzyklus bestimmt. Die Position eines Fußelementes verändert sich im Laufe der Standphase nicht nur in Vorwärtsrichtung, sondern auch in einer Richtung senkrecht dazu. Nach dem Ende der Standphase wird der Fuß angehoben und erst zu Beginn der neuen Standphase wieder auf den Boden aufgesetzt. Soll eine aussagekräftige Höhendifferenz bestimmt werden, ist es von Vorteil, den gleichen Zeitpunkt innerhalb der beiden aufeinanderfolgenden Schrittzyklen zu verwenden, um die Position des Fußes zu diesem jeweiligen Zeitpunkt zu bestimmen und eine Differenz bilden zu können. Vorzugsweise wird die Position während der Standphase bestimmt, wobei insbesondere die Bestimmung der Position in der Standphase von Vorteil ist, da sie sich über einen längeren Zeitraum, nämlich die Dauer der Standphase, nicht verändert und somit sehr genau bestimmt werden kann.

**In** einer bevorzugten Ausgestaltung wird jedoch nicht die Höhendifferenz direkt verwendet, um zu ermitteln, ob der Träger der Prothese bergauf oder bergab geht. Vielmehr wird vorzugsweise ein Steigungswinkel berechnet, der aus der Höhendifferenz und einer Längendifferenz bestimmt wird, die ebenfalls aus der Differenz der Positionen des Fußelementes zu einem bestimmten Zeitpunkt innerhalb zweier aufeinanderfolgender Schrittzyklen bestimmt wurde. Es ergibt sich ein Steigungsdreieck, dessen Steigungswinkel leicht berechnet werden kann. Dieser Steigungswinkel wird vorzugsweise als Kriterium verwendet, um zu bestimmen, ob der Träger der Prothese bergauf oder bergab geht. Dabei wird der Steigungswinkel in einer bevorzugten Ausgestaltung mit einem vorbestimmten Grenzwinkel verglichen. Liegt der Steigungswinkel oberhalb des vorbestimmten Grenzwinkels gilt der Träger der Unterschenkelprothese nicht als bergabgehend. Liegt der Steigungswinkel jedoch unterhalb des vorbestimmten Grenzwinkels, gilt der Träger als bergabgehend.

Vorzugsweise beträgt der Grenzwinkel zwischen 0° und -10°, besonders vorzugsweise -3°.

Vorzugsweise beträgt der vorbestimmte Knöchelwinkelwert zwischen 80° und 100°, besonders bevorzugt 90°. Vorteilhafterweise beträgt der vorbestimmte Unterschenkelwinkelwert zwischen 80° und 100°, vorzugsweise 90°. Vorzugsweise wird der vorbestimmte Knöchelwinkelwert um einen Korrekturwinkel verändert, der sich aus der Absatzhöhe eines Schuhs ergibt, den der Träger trägt. Je höher der Absatz des Schuhs ist, desto größer ist der vorbestimmte Knöchelwinkelwert. Je größer die Absatzhöhe des Schuhs ist, desto mehr verschiebt sich die Nullstellung des Knöchels, also der Winkel zwischen dem Fußelement und dem Unterschenkelelement im entspannten Stehen. Dieser Veränderung der Nullstellung wird durch den Korrekturwinkel Rechnung getragen.

Die Absatzhöhe ist abhängig von der Fußlänge. So würde sich beispielsweise bei einer Fußlänge von 22 cm und einer Absatzhöhe von 3 cm ein Winkel von ungefähr 8° ergeben. Um diesen Winkel, der den Korrekturwinkel bildet, muss der gemessene Winkle oder alternativ der vorbestimmte Knöchelwinkelwert im Sinne eines Offsets korrigiert werden.

Die Erfindung löst die gestellte Aufgabe zudem durch eine Unterschenkelprothese mit einem Fußelementes, einem schwenkbar daran angeordneten Unterschenkelelement und einer verstellbaren Widerstandseinrichtung zum Aufbringen eines Widerstandes gegen eine Verschwenkung des Fußelementes relativ zu dem Unterschenkelelement, wobei die Unterschenkelprothese wenigstens einen Sensor zum Bestimmen einer Höhendifferenz, wenigstens einen Sensor zum Bestimmen des Knöchelwinkels, wenigstens einen Sensor zum Bestimmen des Absolutwinkels des Unterschenkels und eine elektrische Steuerung aufweist, die eingerichtet ist zum Durchführen eines Verfahrens nach einer der vorstehenden Ausführungsformen.

Der Sensor zum Bestimmen des Absolutwinkels des Unterschenkelelementes ist vorzugsweise eine so genannte IMU (Inertial measurement unit - Inertial-Messeinheit), beispielsweise eine Kombination aus Accelerometern und/oder Gyroskopen, mit denen die Orientierung des Unterschenkelelementes im Raum bestimmt und aus dieser der Absolutwinkel des Unterschenkelelementes errechnet wird. Alternativ oder zusätzlich dazu ist der Sensor eingerichtet, den Absolutwinkel des Fußelementes und den Winkel zwischen Fußelement und Unterschenkelelement zu bestimmen, so dass daraus der Absolutwinkel des Unterschenkelelementes errechnet wird.

Die elektrische Steuerung ist vorzugsweise eine elektronische Datenverarbeitungseinrichtung. Besonders bevorzugt weist der wenigstens eine Sensor zum Bestimmen des Knöchelwinkels mindestens einen Absolutwinkelsensor zum Bestimmen des Absolutwinkels des Unterschenkelelementes und mindestens einen Absolutwinkelsensor zum Bestimmen des Absolutwinkels des Fußelementes auf.

Mit Hilfe der beigefügten Abbildungen werden nachfolgend einige Ausführungsbeispiele der vorliegenden Erfindung näher erläutert. Es zeigen:
- Figur 1 -: die schematische Darstellung verschiedener Messgrößen und
- Figur 2a - 2d-: das Erreichen des Blockkriteriums bei unterschiedlichem Untergrund.

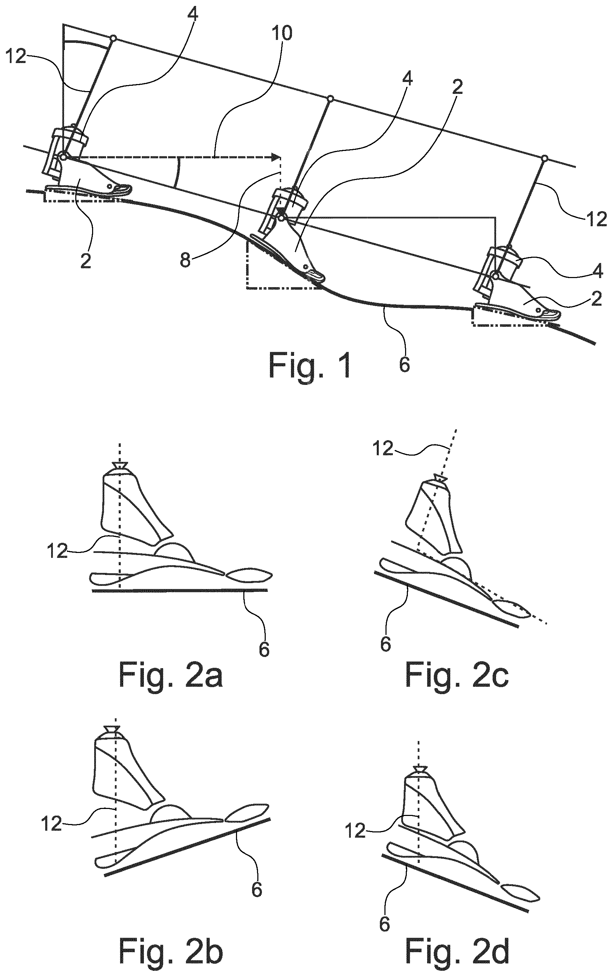

Figur 1 zeigt schematisch einen Anteil einer Unterschenkelprothese mit einem Fußelement 2, an dem ein Unterschenkelelement 4 angeordnet ist. Das Fußelement 2 ist relativ zu dem Unterschenkelelement 4 schwenkbar. Figur 1 zeigt die Position der Unterschenkelprothese in drei aufeinanderfolgenden Standphasen, also den Standphasen dreier aufeinander folgender Schrittzyklen. Das Fußelement 2 ist dabei jeweils mit dem Untergrund 6 in Kontakt. Dieser Untergrund 6 hat jeweils eine lokale Neigung, die der Neigung des Fußelementes 2 an dem jeweiligen Ort des Untergrundes 6 entspricht. Diese lokale Neigung ist durch das unterhalb des Fußelementes 2 dargestellte Dreieck illustriert. Sie muss nicht zum Steuern der Unterschenkelprothese verwendet werden und wird vorzugsweise auch nicht verwendet.

Stattdessen wird im gezeigten Ausführungsbeispiel eine Höhendifferenz 8 und eine Längendifferenz 10 ermittelt, wobei jeweilige Differenz zwischen den Positionen des Fußelementes 2 zwischen zwei aufeinander folgenden Schritten errechnet wird. Beide sind im gezeigten Ausführungsbeispiel zwischen der ersten dargestellten Position und der zweiten dargestellten Position so groß wie zwischen der zweiten dargestellten Position und der dritten dargestellten Position. Die globale Neigung des Untergrundes 6 ist also konstant, obwohl die lokale Neigung stark variieren kann und dies im dargestellten Beispiel auch tut.

In Figur 1 ist die Richtung des Unterschenkelelementes 4 durch eine Linie 12 dargestellt. Der erste Schaltwinkel entspricht einem vorbestimmten Wert des Winkels zwischen der Linie 12 und der lokalen Neigung des Untergrundes 12, die der Richtung des Fußelementes 2 entspricht. Der zweite Schaltwinkel entspricht einer vorbestimmten Orientierung der Linie 12, also einem vorbestimmten Wert des Absolutwinkels des Unterschenkelelementes 4.

Im Folgenden soll der erste Schaltwinkel einem Knöchelwinkelwert von 90° und der zweite Schaltwinkel einem Absolutwinkel von 90°, also eine vertikalen Ausrichtung des Unterschenkelelementes 4 entsprechen.

In Figur 2a ist die Unterschenkelprothese schematisch auf ebenem Untergrund 6 dargestellt. Die Linie 12, die als gestrichelte Linie dargestellt ist, steht senkrecht auf dem Untergrund 6, so dass der erste Schaltwinkel erreicht ist. Die Linie 12 ist zudem auch vertikal ausgerichtet, so dass auch der zweite Schaltwinkel erreicht ist. Der Träger der Prothese geht nicht bergab. Wird dies in dem Verfahren zutreffend erkannt, wird der zweite Schaltwinkel zur Steuerung verwendet, der Widerstand der nicht dargestellten Widerstandseinrichtung also dann erhöht, wenn das Unterschenkelelement 4 senkrecht ausgerichtet ist. Wird in der Steuerung fehlerhafterweise erkannt, dass der Träger bergab geht, wird der zweite erreichte Schaltwinkel zur Steuerung verwendet. Da beide Schaltwinkel gleichzeitig erreicht werden, tritt kein Problem auf und die Erhöhung des Widerstandes findet zum richtigen um vom Träger der Prothese erwarteten Zeitpunkt statt.

**In** Figur 2b ist die Situation dargestellt, in der der Untergrund 6 als ansteigende Rampe ausgebildet ist. Da der Träger auch in diesem Fall nicht bergab geht, wird der zweite Schaltwinkel zum Steuern der Prothese verwendet. Der Widerstand wird also erhöht, wenn der Absolutwinkel des Unterschenkelementes 4, in Figur 2b also die Linie 12, senkrecht steht. Dies ist in Figur 2b gezeigt. Wäre fehlerhafterweise erkannt worden, dass der Träger bergab geht, hätte die Steuerung den Widerstand erhöht, wenn der später erreichte Schaltwinkel erreicht ist. Dies ist in der in Figur 2b gezeigten Situation der zweite Schaltwinkel, so dass in beiden Fällen die Erhöhung des Widerstandes an der vom Benutzer oder Träger der Prothese erwarteten Zeitpunkt erfolgt.

**In** Figur 2c ist die Situation dargestellt, in der der Untergrund 6 als abfallende Rampe ausgebildet ist. Der Träger geht in diesem Fall bergab. Es wird daher der erste Schaltwinkel zum Steuern der Prothese verwendet. Der Widerstand wird also erhöht, wenn der Knöchelwinkel zwischen der Linie 12 des Unterschenkelementes 4 und dem Fußelement 2 90° beträgt. Dies ist in Figur 2c gezeigt. Wäre fehlerhafterweise erkannt worden, dass der Träger nicht bergab geht, hätte die Steuerung den Widerstand erhöht, wenn zweite Schaltwinkel erreicht ist. Die Erhöhung des Widerstandes erfolgte dann also, wenn das Unterschenkelelement und damit dessen Linie 12 senkrecht steht. Dies ist früher als vom Träger erwartet, hat aber keine Sturzgefahr zur Folge, da durch das frühe Erhöhen des Widerstandes ein stabiles Stehen weiterhin gewährleistet bleibt.

Figur 2d zeigt die Situation, in der der Träger der Unterschenkelprothese nicht bergab geht, der Untergrund 6 aber eine lokale Unebenheit aufweist. In diesem Fall wird der zweite Schaltwinkel zum Steuern der Prothese verwendet, der in Figur 2d erreicht ist.

### Bezugszeichenliste

- 2: Fußelement
- 4: Unterschenkelelement
- 6: Untergrund
- 8: Höhendifferenz
- 10: Längendifferenz
- 12: Linie

## Patentansprüche

1. Verfahren zum Steuern einer Unterschenkelprothese, die ein Fußelement (2), ein schwenkbar daran angeordnetes Unterschenkelelement (4) und eine verstellbare Widerstandseinrichtung zum Aufbringen eines Widerstandes gegen eine Verschwenkung des Fußelementes (2) relativ zu dem Unterschenkelelement (4) aufweist, wobei
- ein erster Schaltwinkel definiert ist als ein vorbestimmter Knöchelwinkelwert zwischen dem Unterschenkelelement (4) und dem Fußelement (2),
- ein zweiter Schaltwinkel definiert ist als ein vorbestimmter Unterschenkelwinkelwert des Absolutwinkels des Unterschenkelelements (4)
wobei bei dem Verfahren
- zumindest auch aus einer Höhendifferenz (8) zwischen der Position des Fußelementes (2) in einem Schrittzyklus und der Position des Fußelementes (2) in dem vorherigen Schrittzyklus bestimmt wird, ob der Träger der Unterschenkelprothese bergab geht, und
- der Widerstand der Widerstandseinrichtung auf einen vorbestimmten Widerstandswert erhöht wird, wenn ein Blockkriterium erfüllt ist,
wobei das Blockkriterium erfüllt ist,
wenn bestimmt wurde, dass der Träger nicht bergab geht, und der zweite Schaltwinkel erreicht ist, oder
wenn bestimmt wurde, dass der Träger bergab geht und der in dem Schrittzyklus später erreichte Schaltwinkel erreicht ist.

2. Verfahren nach Anspruch 1, **dadurch gekennzeichnet, dass** der vorbestimmte Widerstandswert so groß ist, dass eine weitere Verschwenkung des Fußelementes (2) relativ zum Unterschenkelelement (4) verhindert wird.

3. Verfahren nach Anspruch 1 oder 2, **dadurch gekennzeichnet, dass** die Höhendifferenz (8) zwischen der Position des Fußelementes (2) in der Standphase der beiden Schrittzyklen bestimmt wird.

4. Verfahren nach einem der vorstehenden Ansprüche, **dadurch gekennzeichnet, dass** aus einem Steigungswinkel, der aus der Höhendifferenz (8) und einer Längendifferenz (10) zwischen den Positionen des Fußelementes (2) bestimmt wird, bestimmt wird, ob der Träger der Unterschenkelprothese bergab geht.

5. Verfahren nach Anspruch 4, **dadurch gekennzeichnet, dass** der Träger dann als bergabgehend angesehen wird, wenn der Steigungswinkel kleiner als ein Grenzwinkel ist, der vorzugsweise zwischen 0° und -10° liegt und besonders vorzugsweise -3° beträgt.

6. Verfahren nach einem der vorstehenden Ansprüche, **dadurch gekennzeichnet, dass** der vorbestimmte Knöchelwinkelwert zwischen 80° und 100°, vorzugsweise 90° beträgt.

7. Verfahren nach einem der vorstehenden Ansprüche, **dadurch gekennzeichnet, dass** der vorbestimmte Unterschenkelwinkelwert zwischen 80° und 100°, vorzugsweise 90° beträgt.

8. Unterschenkelprothese mit einem Fußelement (2), einem schwenkbar daran angeordneten Unterschenkelelement (4) und einer verstellbaren Widerstandseinrichtung zum Aufbringen eines Widerstandes gegen eine Verschwenkung des Fußelementes (2) relativ zu dem Unterschenkelelement (4), wobei die Unterschenkelprothese wenigstens einen Sensor zum Bestimmen einer Höhendifferenz (8), wenigstens einen Sensor zum Bestimmen des Knöchelwinkels, wenigstens einen Sensor zum Bestimmen des Absolutwinkels des Unterschenkelelements (4) und eine elektrische Steuerung aufweist, die eingerichtet ist zum Durchführen eines Verfahrens nach einem der vorstehenden Ansprüche.

9. Unterschenkelprothese nach Anspruch 8, **dadurch gekennzeichnet, dass** der wenigstens eine Sensor zum Bestimmen des Knöchelwinkels einen Absolutwinkelsensor zum Bestimmen des Absolutwinkels des Unterschenkelelementes (4) und einen Absolutwinkelsensor zum Bestimmen des Absolutwinkels des Fußelementes (2) aufweist.

## Claims

1. A method for controlling a lower leg prosthesis comprising a foot element (2), a lower leg element (4) pivotably arranged thereon and an adjustable resistance device for applying a resistance against a swivelling of the foot element (2) relative to the lower leg element (4), wherein
- a first switching angle is defined as a predetermined ankle angle value between the lower leg element (4) and the foot element (2),
- a second switching angle is defined as a predetermined lower leg angle value of the absolute angle of the lower leg element (4),
the method comprising the steps:
- determining, at least also on the basis of a height difference (8) between the position of the foot element (2) in a step cycle and the position of the foot element (2) in the previous step cycle, whether the wearer of the lower leg prosthesis is walking downhill, and
- increasing the resistance of the resistance device to a predetermined resistance value when a block criterion is met,
wherein the block criterion is met
if it has been determined that the wearer is not walking downhill and the second switching angle is reached, or
if it has been determined that the wearer is walking downhill and the switching angle reached later in the step cycle is reached.

2. The method according to claim 1, **characterized in that** the predetermined resistance value is big enough to prevent a further swivelling of the foot element (2) relative to the lower leg element (4).

3. The method according to claim 1 or 2, **characterized in that** the height difference (8) between the position of the foot element (2) in the stance phase of the two step cycles is determined.

4. The method according to one of the preceding claims, **characterized in that** a pitch angle, which is determined from the height difference (8) and a length difference (10) between the positions of the foot element (2), is used to determine whether the wearer of the lower leg prosthesis is walking downhill.

5. The method according to claim 4, **characterized in that** the wearer is deemed to be walking downhill if the pitch angle is smaller than a limit angle, which is preferably between 0° and -10° and particularly preferably is -3°.

6. The method according to one of the preceding claims, **characterized in that** the predetermined ankle angle value is between 80° and 100°, preferably 90°.

7. The method according to one of the preceding claims, **characterized in that** the predetermined lower leg angle value is between 80° and 100°, preferably 90°.

8. A lower leg prosthesis with a foot element (2), a lower leg element (4) pivotably arranged thereon and an adjustable resistance device for applying a resistance against a swivelling of the foot element (2) relative to the lower leg element (4), wherein the lower leg prosthesis comprises at least one sensor for determining a height difference (8), at least one sensor for determining an ankle angle, at least one sensor for determining the absolute angle of the lower leg element (4), and an electrical control unit that is configured to conduct a method according to one of the preceding claims.

9. The lower leg prosthesis according to claim 8, **characterized in that** the at least one sensor for determining the ankle angle comprises an absolute angle sensor for determining the absolute angle of the lower leg element (4) and an absolute angle sensor for determining the absolute angle of the foot element (2).

## Revendications

1. Procédé pour commander une prothèse de jambe comportant un élément de pied (2), un élément de jambe (4) monté de manière pivotante sur celui-ci, et un dispositif de résistance réglable pour exercer une résistance au pivotement de l'élément de pied (2) par rapport à l'élément de jambe (4), sachant que
- un premier angle de commutation est défini comme une valeur prédéterminée d'angle de cheville entre l'élément de jambe (4) et l'élément de pied (2),
- un deuxième angle de commutation est défini comme une valeur prédéterminée d'angle de jambe de l'angle absolu de l'élément de jambe (4), procédé dans lequel
- il est déterminé si le porteur de la prothèse de jambe descend une pente au moins également à partir d'une différence de hauteur (8) entre la position de l'élément de pied (2) dans un cycle de marche et la position de l'élément de pied (2) dans le cycle de marche précédent, et
- la résistance du dispositif de résistance est augmentée à une valeur de résistance prédéterminée si un critère bloc est rempli,
le critère bloc étant rempli
lorsqu'il a été déterminé que le porteur ne descend pas une pente et que le deuxième angle de commutation est atteint, ou
lorsqu'il a été déterminé que le porteur descend une pente et que l'angle de commutation, atteint plus tard dans le cycle de marche, est atteint.

2. Procédé selon la revendication 1,
**caractérisé en ce que** la valeur de résistance prédéterminée est suffisamment grande pour empêcher une poursuite du pivotement de l'élément de pied (2) par rapport à l'élément de jambe (4).

3. Procédé selon la revendication 1 ou 2,
**caractérisé en ce que** la différence de hauteur (8) entre les positions de l'élément de pied (2) est déterminée dans la phase d'appui des deux cycles de marche.

4. Procédé selon l'une des revendications précédentes,
**caractérisé en ce qu'**il est déterminé si le porteur de la prothèse de jambe descend une pente sur la base d'un angle d'inclinaison déterminé à partir de la différence de hauteur (8) et d'une différence de longueur (10) entre les positions de l'élément de pied (2).

5. Procédé selon la revendication 4,
**caractérisé en ce que** le porteur est alors considéré comme descendant une pente si l'angle d'inclinaison est inférieur à un angle limite qui est de préférence compris entre 0° et -10° et qui est de préférence encore de -3°.

6. Procédé selon l'une des revendications précédentes,
**caractérisé en ce que** la valeur prédéterminée d'angle de cheville est comprise entre 80° et 100° et est de préférence de 90°.

7. Procédé selon l'une des revendications précédentes,
**caractérisé en ce que** la valeur prédéterminée d'angle de jambe est comprise entre 80° et 100° et est de préférence de 90°.

8. Prothèse de jambe comprenant un élément de pied (2), un élément de jambe (4) monté de manière pivotante sur celui-ci, et un dispositif de résistance réglable pour exercer une résistance au pivotement de l'élément de pied (2) par rapport à l'élément de jambe (4), la prothèse de jambe comprenant au moins un capteur pour déterminer une différence de hauteur (8), au moins un capteur pour déterminer l'angle de la cheville, au moins un capteur pour déterminer l'angle absolu de l'élément de jambe (4), et une commande électrique qui est conçue pour mettre en œuvre un procédé selon l'une des revendications précédentes.

9. Prothèse de jambe selon la revendication 8,
**caractérisée en ce que** ledit au moins un capteur pour déterminer l'angle de la cheville comprend un capteur d'angle absolu pour déterminer l'angle absolu de l'élément de jambe (4) et un capteur d'angle absolu pour déterminer l'angle absolu de l'élément de pied (2).
